# NEW EUROPEAN PATENT SPECIFICATION

(11) **EP 1 115 432 B2**
(45) Date of publication and mention of the opposition decision: **19.12.2007**
(45) Mention of the grant of the patent: 10.12.2003
(21) Application number: 99944884.8
(22) Date of filing: 13.09.1999
(51) Int. Cl.: A61L 15/28, A61L 15/32, A61L 15/44

(54) **DERMAL SCAFFOLD USING NEUTRALIZED CHITOSAN SPONGE OR NEUTRALIZED CHITOSAN/COLLAGEN MIXED SPONGE**
DERMALES GERÜST AUF DER GRUNDLAGE EINES NEUTRALISIERTEN CHITOSAN-SCHWAMMES ODER EINES NEUTRALSIERTEN GEMISCHTEN CHITOSAN-KOLLAGEN-SCHWAMMES
ECHAFAUDAGE DERMIQUE UTILISANT UNE EPONGE A CHITOSANE NEUTRALISEE OU UNE EPONGE MIXTE A CHITOSANE/COLLAGENE NEUTRALISEE

(30) Priority: 24.09.1998 KR 9839576
(43) Date of publication of application: 18.07.2001
(73) Proprietor: Korea Atomic Energy Research Institute, Daejeon 305-353 (KR)
(72) Inventor: SON, Young-Sook;, Dongjak-ku, Seoul 156-092 (KR); YOUN, Yong-Ha, Bupyung-ku, Incheon 403-132 (KR); HONG, Seok-Il;, Nowon-ku, Seoul 139-220 (KR); LEE, Seung-Hoon;, Nowon-ku, Seoul 139-220 (KR); GIN, Yong-Jae, Nowon-ku, Seoul 139-230 (KR)
(74) Representative: Kaiser, Jürgen
(86) International application number: PCT/KR1999/000540
(87) International publication number: WO 2000/016817

(56) References cited:
- EP-A1- 0 296 078
- EP-A1- 0 562 864
- EP-A2- 0 702 081
- WO-A1-97/44070
- DE-A1- 3 444 746
- US-A- 5 041 138
- PATENT ABSTRACTS OF JAPAN & JP 09 173 362 A (MENICON CO LTD) 08 July 1997

## Description

### TECHNICAL FIELD

The present invention relates to a dermal scaffold and a bioartificial dermis using the same. More specifically, the present invention relates to the dermal scaffold and the bioartificial dermis comprising a sponge, consisting of neutralized chitosan neutralized chitosan/collagen mixed sponge according to claim 4 or neutralized chitosan/collagen mixed sponge wherein the weight ratio of collagen to chitosan is in the range of 1:8 to 1:2 based on the final product containing chitosan fabrics, which are extremely useful for wound healing therapy.

### BACKGROUND ART

Various kinds of ordinary dressings for wound healing have been hitherto developed and conveniently used, but in most cases, they have been used only for the prevention of infection or dehydration.

Recently, acellular artificial skins or cell-based bioartificial skins have been developed and marketed by many biotechnological companies. As the acellular artificial skins, for example, an acellular collagen-glycosaminoglycan matrix bonded to a thin silicone membrane (INTEGRAft^{™}, Interga LifeSciences Co.) and dehydrorothermally cross-linked composites of fibrillar and denatured collagens (Terudermis ^{™}, Terumo Co.) are now commercially available. However, such products are very expensive by incorporation of biomaterials such as collagen and thus, have difficulty in clinical trials on broad wound sites, *e.g*. burns.

As the cell-based bioartificial skins, Advanced Tissue Sciences, Inc. (La Jolla, California) developed a skin replacement product composed of a thin biodegradable mesh framework onto which human dermal fibroblasts are seeded, for use in treating diabetic foot ulcers (Dermagraft-TC^{™}). In addition, epidermal cell sheet for partial-thickness wound (Acticel™, Biosurface Technology, Inc.), composite grafts of cultured keratinocytes and fibroblasts on a collagen glycosaminoglycan matrix (Apligraft^{™}, Organogenesis, Inc.) and a skin replacement product derived from human cadaver skin (Alloderm^{™}, Lifecell), etc. were developed.

The cell-based bioartificial skins were prepared by primary culture of human dermal fibroblasts and keratinocytes followed by 3-dimensional culture (3-D culture, raft culture) of the cultured cells in hydrated collagen. They had considerably good wound healing and scar reducing effects in clinical trials on burn or plastic surgery patients. However, they still have problems in that they are too expensive due to incorporation of collagen (e.g. Dermagraft-TC: 2,000 $/ 10 × 10 cm, Terudermis: 1,500 $) and are limited in their uses as grafts due to a low rigidity of hydrated collagen gel. Therefore, there still exists an important demand for development of polymers with good biocompatibility and biodegradability which can successfully replace collagen and is also suitable for use as a scaffold.

A polysaccharide sponge characterized by having: (i) an average pore size in the range between about 10 mum to about 300 mum; (ii) an average distance between the pores being the wall thickness of the pores in the range between about 5 mum to about 270 mum; and (iii) an E-modulus of elasticity being a measure of the rigidity of the sponge in the range of about 50 kPa to about 500 kPa is disclosed in WO-A1-9744070.

Chitosan is a cationic polysaccharide composed of glucosamine and N-acetylglucosamine residues with a 1,4-β-linkage. Chitosan can be prepared by deacetylation of chitin from crabs or other sources. Chitin is slowly degraded *in vivo* and thus, chitin and its degradation products are natural and safe. In pharmaceutical area, chitosan has been used as a vehicle for the sustained release of drugs (Hou et al., Chem Pharm Bull 1985; 33(9): 3986-3992). Chitin as such has been woven into fabrics and used as dressings for wound healing. However, neutralized chitosan has never been reported to be used as a dermal scaffold.

### DISCLOSURE OF THE INVENTION

Accordingly, in order to solve the above-mentioned problems involved in the prior art, an object of the present invention is to provide a novel dermal scaffold for wound healing with excellent biocompatibility and biodegradability which is not only cost-effective but also convenient to manipulate owing to the improved rigidity, and the dermal scaffold further comprising basic fibroblast growth factor (bFGF), fibronectin, etc. for accelerating wound healing.

Another object of the present invention is to provide a bioartificial dermis wherein human fibroblasts are loaded onto the dermal scaffold, particularly useful for healing of broad wound sites such as burns.

In order to attain the above-described objects, one aspect of the present invention provides a dermal scaffold comprising a sponge, consisting of neutralized chitosan neutralized chitosan/collagen mixed sponge, according to claim 4 or neutralized chitosan/collagen mixed sponge wherein the weight ratio of collagen to chitosan is in the range of 1:8 to 1:2 based on the final product containing chitosan fabrics. The dermal scaffold preferably further comprises one or more components selected from the group consisting of fibronectin, basic fibroblast growth factor, epidermal growth factor and transforming growth factor-β.

Another aspect of the present invention provides a bioartificial dermis wherein human fibroblasts are loaded onto neutralized chitosan, neutralized chitosan/collagen mixed sponge or neutralized chitosan/collagen mixed sponge containing chitosan fabrics according to the present invention.

### BRIEF DESCRIPTION OF THE DRAWINGS

Fig 1 is a light microscopy of prepared chitosan and collagen sponges (magnification × 200);
Fig 2 is a light microscopy of chitosan sponge with cultured human fibroblasts (magnification × 200);
Fig 3 is a light microscopy of neutralized chitosan sponge comprising human fibroblasts containing bFGF (50 ng/mℓ) or no bFGF (magnification × 200);
Fig 4 shows histology of the cross-sections on neutralized chitosan sponge (A) and neutralized chitosan/collagen mixed sponge (B) with cultured human fibroblasts for 4 weeks *in vitro* (magnification × 200);
Fig 5 shows chitosan sponge grafts on the full-thickness excision in the Balb/c mouse;
Fig 6 shows graft sites in Balb/c mouse 4 weeks after grafting;
Fig 7 shows gross histology of control biopsied from the dorsolateral region of Balb/c mouse 10 day after grafting;
Fig 8 shows gross histology of graft site biopsied from the dorsolateral region of Balb/c mouse grafted with neutralized chitosan sponge 10 days after grafting;
Fig 9 shows gross histology of graft site biopsied from the dorsolateral region of Balb/c mouse grafted with neutralized chitosan + bFGF (50 ng/mℓ) sponge 10 days after grafting;
Fig 10 shows gross histology of graft site biopsied from the dorsolateral region of Balb/c mouse grafted with neutralized chitosan + type-Ip collagen sponge 10 days after grafting; and
Fig 11 is a graph showing viability assay result of human dermal fibroblasts treated with chitosan derivates.

### BEST MODE FOR CARRYING OUT THE INVENTION

Hereinafter, the present invention will be more specifically explained.

The present inventors unexpectedly found that spongy scaffold could be obtained by neutralization of a chitosan solution with an alkaline solution followed by lyophilization of the neutralized chitosan solution. The obtained neutralized chitosan sponge is cost-effective compared to the art-known collagen sponge products and has the improved rigidity to be convenient to manipulate. Further, it can be grafted onto the wounded tissue thereby to exhibit excellent wound healing effect, and therefore, would be extremely useful as dressings for wound healing.

In order to prepare the dermal scaffold comprising neutralized chitosan sponge according to the present invention, first, a chitosan solution is mixed with an alkaline solution to obtain a neutralized chitosan solution. According to the present invention, the chitosan solution is preferably prepared by dissolving chitosan in 1% acetic acid solution at concentrations of 1 to 2 w/v%. As the alkaline solution for neutralization, a mixed solution of reconstruction buffer (2.2 g of NaHCO₃, 4.77 g of HEPES (200 mM) / 100 mℓ of 0.05 N NaOH) and 10 × medium free of NaHCO₃ (DMEM:F12 = 3:1, Gibco BRL. DMEM-Cat. No. 12800-058, F12-Cat, No. 21700-026) is preferably used. The neutralized chitosan solution is frozen at a low temperature, particularly, at -70 °C for one day or under liquid nitrogen (at about -140 °C) and then, lyophilized (-42 °C) to obtain a spongy dermal scaffold. The obtained dermal scaffold is sterilized and then applied to wound sites. In the present invention, γ -ray or ultraviolet irradiation or 70% ethanol soaking is preferably employed for sterilization.

In order to increase biocompatibility of the neutralized chitosan sponge according to the present invention, neutralized chitosan/collagen mixed sponge according to claim 4 may be employed. The neutralized chitosan/collagen mixed sponge may be prepared by mixing the neutralized chitosan solution prepared as above with a neutralized type-Ip collagen (atelomeric collagen) solution prepared according to the substantially same procedure as the neutralized chitosan solution. The mixing ratio is adjusted for a weight ratio of collagen to chitosan to be in the range of 1:8 to 1:2 based on a final product. However, it may be appropriately varied considering the desired rigidity and histological characteristics of the wounded tissue. Since collagen is likely to form a gel at room temperature, preparation and neutralization of the collagen solution is preferably performed at 4 °C or less.

In order to increase tensility of the neutralized chitosan sponge, neutralized chitosan/collagen mixed sponge containing chitosan fabrics according to the present invention may be prepared. More specifically, chitosan fibers may be woven into fabrics according to any conventional method. Onto the woven chitosan fabrics is placed the neutralized chitosan solution, and neutralized chitosan sponge containing chitosan fabrics is prepared according to the above-described procedure. Then, to increase attachment of human fibroblasts, the prepared sponge is coated with the neutralized type-Ip collagen solution to obtain chitosan/collagen mixed sponge containing chitosan fabrics.

Neutralized chitosan sponge, neutralized chitosan/collagen mixed sponge or neutralized chitosan/collagen mixed sponge containing chitosan fabrics according to the present invention may further comprise fibronectin, basic fibroblast growth factor, epidermal growth factor, transforming growth factor-β, etc. In the present invention, the sponge preferably comprises 50 to 500 ng/mℓ of fibronectin or 10 to 100 ng/mℓ of basic fibroblast growth factor.

In addition, a bioartificial dermis prepared by loading human fibroblasts onto the sponge is useful for wound healing by being grafted onto the relatively broad wound sites. The bioartificial dermis may be prepared by loading 1∼5 × 10⁵ cells/cm² onto the sponge followed by culturing it under conditions suitable for attachment and proliferation of human fibroblasts. However, for emergency, it will be allowed to graft the bioartificial dermis comprising human fibroblasts cultured for just one day.

In the present neutralized chitosan sponge or neutralized chitosan/collagen mixed sponge, any shapes such as round, square, etc. may be allowed and size and thickness thereof may be appropriately varied in accordance with application sites and particularly, it may be molded into various shapes, *e.g*. nose, ear, etc.

### EXAMPLES

This invention will be better understood from the following examples.

### Examples 1 : Preparation of a dermal scaffold

### (1) Preparation of a dermal scaffold comprising chitosan sponge

Weighed amounts of chitosan (Fluka, medium MW; ∼400,000) were dissolved in 1 v/v% acetic acid to give 2 w/v% solutions. The solutions were stirred for 1 hour at room temperature and then, incubated overnight at room temperature to remove entrapped air bubbles from the solutions in a dust-free atmosphere. 20 mℓ of the solutions were added to plastic petri dishes having a diameter of 100 mm (to prepare round sponge having a diameter of 100 mm) and allowed to stand overnight at - 70 °C. The frozen solutions were lyophilized in a freeze-dryer for 48 hours to obtain a chitosan sponge. The sponge was washed with phosphate buffered saline three times. Subsequently, the obtained chitosan sponge was frozen and lyophilized again under the same condition as above and then, sterilized by γ-ray irradiation (20 Kgy/5hrs, γ-ray source; Co⁶⁰). 30 W Ultraviolet was irradiated on each surface of this sponge at a distance of 30 cm for 1 hour to make the sponge insoluble in water. Thus, a spongy dermal scaffold having a thickness of 3.0∼4.0 mm was obtained.

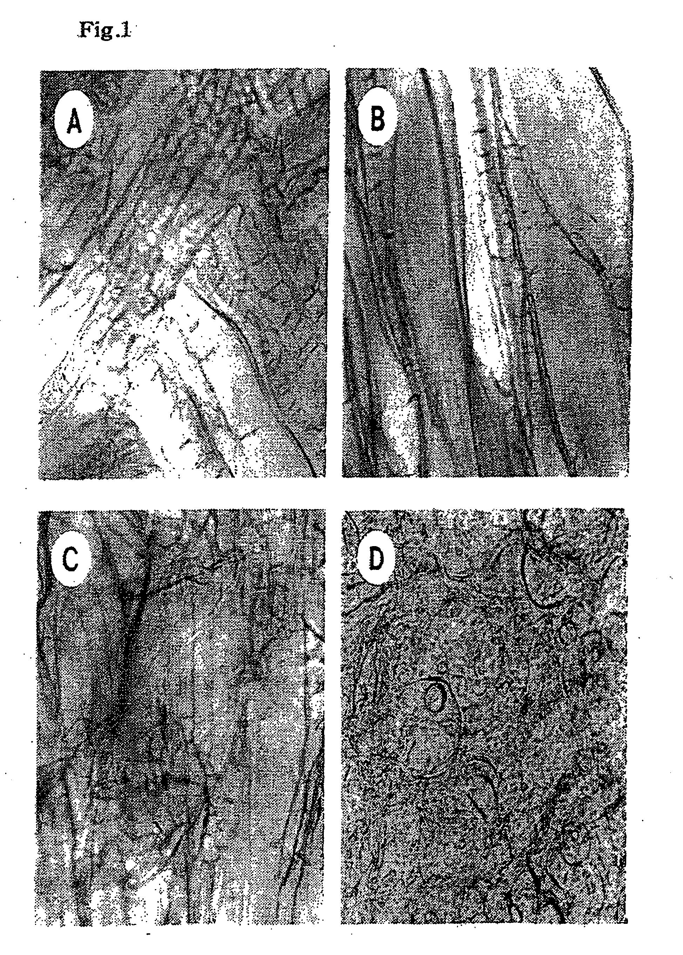

The obtained chitosan sponge was smooth and flexible and composed of chitosan fibers to be porous. Fig 1 shows the semi-transparent chitosan sponge under a phase-contrast light microscopy (A).

### (2) Preparation of a dermal scaffold comprising neutralized chitosan sponge

In order to prepare a neutralized chitosan sponge, 2 w/v% chitosan solution dissolved in acetic acid, reconstruction buffer (2.2 g of NaHCO₃, 4.77 g of HEPES (200 mM)/ 100 mℓ of 0.05 N NaOH) and 10 × medium free of NaHCO₃ (DMEM:F12 = 3:1, Gibco BRL. DMEM-Cat. No. 12800-058, F12-Cat. No. 21700-026) were mixed in a ratio of 8:1:1 to obtain a neutralized chitosan solution. Neutralized chitosan sponge was prepared using the neutralized chitosan solution according to the same procedure as example 1 (1). Fig 1 shows the semi-transparent neutralized chitosan sponge under a phase-contrast light microscopy (B).

### (3) Preparation of a dermal scaffold comprising neutralized chitosan/collagen mixed sponge

In order to prepare a neutralized collagen solution, type-Ip collagen (3 mg/mℓ at a pH of 3.0; Cell matrices, Gelatin Corp., Osaka, Japan, swine collagen free of telomeric portion by treatment with pepsin) solution, reconstruction buffer and 10× medium free of NaHCO₃ were mixed in a ratio of 8:1:1 and then, the mixed solution was neutralized. Neutralization and manipulation of the collagen solution were performed at 4 °C or less. The obtained neutralized collagen solution and the neutralized chitosan solution prepared in example 1 (2) were mixed in a ratio of 1:1 (v/v) to obtain a solution for preparation of a chitosan/collagen mixed sponge (as a final concentration, 8 mg/mℓ of chitosan, 1.2 mg/mℓ of collagen). A dermal scaffold was prepared using the chitosan/collagen mixed solution according to the substantially same procedure as examples 1 (1) and (2). Fig 1 shows the semi-transparent neutralized chitosan/collagen sponge (C) and type-Ip collagen sponge (D) under a phase-contrast light microscopy.

### (4) Preparation of a neutralized chitosan/collagen mixed sponge containing chitosan fabrics

In order to increase tensility of the neutralized chitosan sponge, neutralized chitosan/collagen mixed sponge containing chitosan fabrics was prepared as follows.

First, chitosan fibers were woven into fabrics according to any conventional method. Onto the woven chitosan fabrics was placed the neutralized chitosan solution prepared in example 1 (2) and neutralized chitosan sponge containing chitosan fabrics was prepared according to the substantially same procedure as example 1 (1). Then, to increase attachment of human fibroblasts, the prepared sponge was coated with the neutralized type-Ip collagen solution prepared in example 1 (3) to obtain chitosan/collagen mixed sponge containing chitosan fabrics.

The neutralized chitosan/collagen mixed sponge containing chitosan fabrics according to the present example has the improved tensility and may be conveniently manipulated and stored.

### (5) Preparation of a dermal scaffold comprising fibronectin and basic fibroblast growth factor

To the solutions for preparing chitosan, neutralized chitosan and neutralized chitosan/collagen mixed sponge prepared in examples 1 (1) to (3) was added fibronectin or basic fibroblast growth factor at concentrations of 5, 50 and 500 ng/mℓ, respectively. In the above case, a final sponge comprised 0.67 ng/ 8 mm-diameter sponge of fibronectin and 0.33 ng/ 8 mm-diameter sponge of basic fibroblast growth factor, respectively.

A dermal scaffold was prepared using the mixed solution according to the substantially same procedure as examples 1 (1) to (3) except for performing the sterilization by γ-ray irradiation (5 Kgy/ 5hrs, γ-ray source; Co⁶⁰).

### Example 2 : Preparation of a bioartificial dermis

### (1) Primary pure culture of human dermal fibroblasts

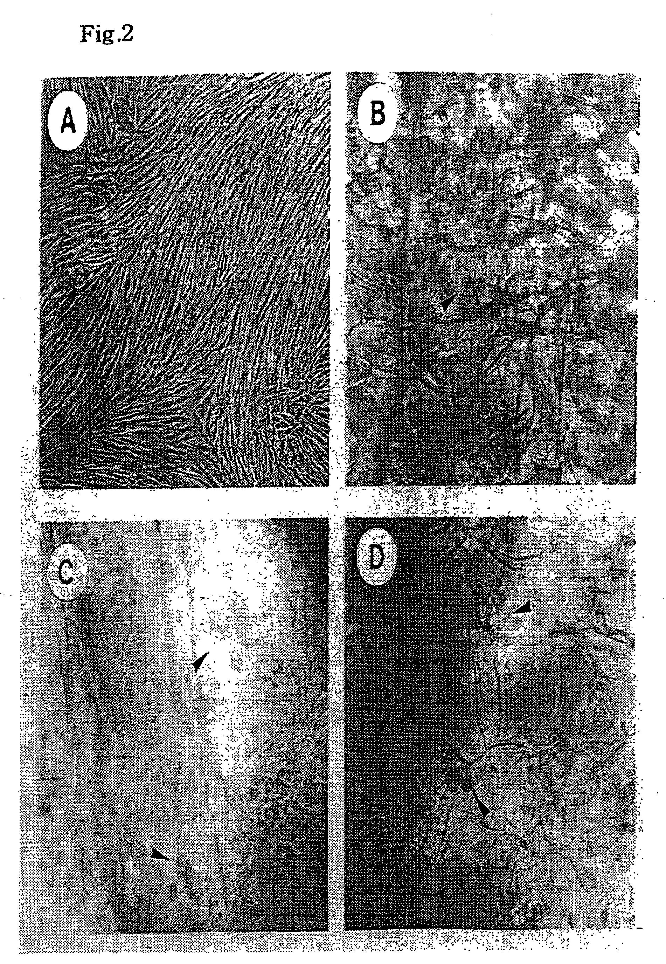

Human dermal fibroblasts (HDFs) were isolated from neonatal foreskin obtained aseptically after circumcision. Dermis was separated from epidermis by incubation in 0.25% trypsin/ 0.02% EDTA solution for one hour at 37 °C. Dermis was minced and digested with 0.35% collagenase B solution for one hour at 37 °C. The obtained cells were washed several times to remove collagenase and then, the cells were suspended in DMEM medium supplemented with 10% fetal bovine serum. Fibroblasts were cultured in a tissue culture flask at 37°C, 5% CO₂, in a humidified incubator. After reaching a confluency of 80∼ 90%, the cells were subcultured using 0.1% trypsin solution. The standard number of the suspended cells in the subculture was 1 × 10⁶ cells/ 100 mm dish. The cells were stored in a cryopreserved solution (DMEM 50%, fetal bovine serum 40%, DMSO 10%). Before preparation for loading onto a dermal scaffold, the cells were thawed and recultured. To prepare a bioartificial dermis, the cryopreserved cells were diluted with phosphate buffered saline and centrifuged three times. Then, the cells were washed, suspended in the medium and recultured. Fig 2 shows the primary pure cultured human dermal fibroblasts (A).

### (2) Attachment of human dermal fibroblasts into a dermal scaffold

In a laminar-flow hood, the sponges were punched at diameters of 8 mm and 100 mm and placed in a culture dish (24 wells, a diameter of 150 mm). To prepare round sponge having a diameter of 8 mm, 1 × 10⁵ of viable cells (determined by tryphan blue solution) were diluted with minimum volume of DMEM medium and placed onto the sponge. Then, it was allowed to stand at 37 °C, 5% CO₂ for 4 hours and 50 µℓ of DMEM medium was further added thereto. 24 hours after the addition, 1 mℓ of the medium was added to each well and the cells were cultured. The artificial dermis was maintained under the same condition as above for 4 weeks to reach a constant confluency. All the cells and tissue matrices formed adhering to the sponge were observed under a phase-contrast light microscopy. The medium was replaced three times a week.

Fig 2 shows neutralized chitosan sponge comprising human fibroblasts (B), neutralized chitosan sponge comprising fibronectin (C) and neutralized chitosan/type-Ip collagen mixed sponge comprising fibronectin (D), respectively.

### (3) Evaluation of human dermal fibroblasts viability and growth in neutralized chitosan and neutralized chitosan/collagen mixed sponges in vitro

Viability of cells after enzymatically dissociation form culture dishes was about 95-100% as estimated with the vital stain tryphan blue. These cells were inoculated into chitosan and neutralized chitosan sponges at a seeding concentration of 1 × 10⁵ cells/ sponge (a diameter 8 mm). 1 week after inoculation on chitosan and neutralized chitosan sponges, these sponges were trypsinized, and viable dislodged cells were counted under a light microscopy. The following table 1 shows viable fibroblast cell numbers in chitosan sponges 1 week after inoculation.

**Table 1.**

| Fibroblast cell numbers in chitosan sponges 1 week after inoculation (1×10⁴ cells). | | |
|---|---|---|
| | chitosan sponge | neutralized chitosan sponge |
| control | 6.0±2.0 | 42.7±10.6 |
| + fibronectin (100 ng/mℓ) | 22.7±3.4 | 93.3±6.2 |
| + bFGF (50 ng/mℓ) | 68.4±16.1 | 312.9±63.0 |
| + type-Ip collagen (1.5 mg/mℓ) | - | 332.0±44..0 |
| * the above experimental data were obtained by SEM(±) (- shows no detection; n=4) | | |

Compared to the first seeding concentration, viable cells grown in chitosan sponge decreased about 40%, but increased about 427% in neutralized chitosan sponge. In the estimation of viability on fibronectin (100 ng/mℓ) mixed chitosan and neutralized chitosan sponges, viable cells markedly increased approximately 5.5 and 2 times compared to those in chitosan and neutralized chitosan sponges, respectively. The number of cells grown in bFGF (50 ng/mℓ) mixed chitosan and neutralized chitosan sponges significantly increased about 17 and 9 times compared to that in both chitosan and neutralized chitosan sponges, and also, in type-Ip collagen (1.5 mg/mℓ) mixed neutralized chitosan sponge increased about 9 times compared to that in neutralized chitosan sponge.

### (4) Cytological/biochemical analysis of a bioartificial dermis

The bioartificial dermis with human dermal fibroblasts cultured for 1 week was removed from the culture dish and then washed with phosphate buffered saline three times. The dermis was exposed to 0.25% trypsin solution for 10 minutes to isolate fibroblasts from the bioartificial dermis. The dermis was agitated in a petri dish and then washed with DMEM medium to obtain a suspension. The resulting suspension was added to a plastic test tube containing 10% fetal bovine serum and centrifuged for 5 minutes. 1 mℓ of DMEM medium was added to cell pellet to obtain a suspension. 100 mℓ of the resulting suspension was stained with a tryphan blue solution and viable cells were counted under a light microscopy. The results are shown in the following table 2.

**Table 2.**

| Viable human dermal fibroblast numbers in chitosan sponges one week after attachment of the cells onto the sponges (1 × 10⁴ cells) | | | |
|---|---|---|---|
| | | neutralized chitosan sponge | neutralized chitosan/collagen mixed sponge (1.2 mg/mℓ of Type-Ip collagen) |
| control | | 8.5±1.2 | 41.5±1.9 |
| + bFGF | 5 ng/mℓ | 18.0±1.4 | 37.0±2.1 |
| | 50 ng/mℓ | 33.5±2.1 | 88.0±2.7 |
| | 500 ng/mℓ | 24.0±3.4 | 98.0±3.5 |
| + fibronectin | 5 ng/mℓ | 16.0±2.2 | 44.3±1.2 |
| | 50 ng/mℓ | 23.5±0.9 | 58.3±2.7 |
| | 500 ng/mℓ | 20.0±1.7 | 58.0±3.2 |
| * the above experimental data were obtained by SEM(±) (n=4) | | | |

As shown in the above table 2, one week after loading the cells onto the sponge, viable cells in the neutralized chitosan/type-Ip collagen mixed sponge increased 5 times compared to those in the neutralized chitosan sponge. Viable cells in the neutralized chitosan sponge comprising 5, 50 and 500 ng/mℓ of bFGF increased 2, 4 and 3 times compared to those in the neutralized chitosan sponge comprising no bFGF, respectively. Viable cells in the neutralized chitosan sponge comprising 5, 50 and 500 ng/mℓ of fibronectin increased 2, 4, 3 times compared to those in the neutralized chitosan sponge comprising no fibronectin, respectively.

Additionally, viable cells in the neutralized chitosan/collagen mixed sponge comprising 5, 50 and 500 ng/mℓ of bFGF increased 4.5, 10 and 11.5 times compared to those in the neutralized chitosan/collagen mixed sponge comprising no bFGF, respectively. Viable cells in the neutralized chitosan/collagen mixed sponge comprising 5, 50 and 500 ng/-mℓ of fibronectin increased 5, 7 and 7 times compared to those in the neutralized chitosan/collagen mixed sponge comprising no fibronectin, respectively.

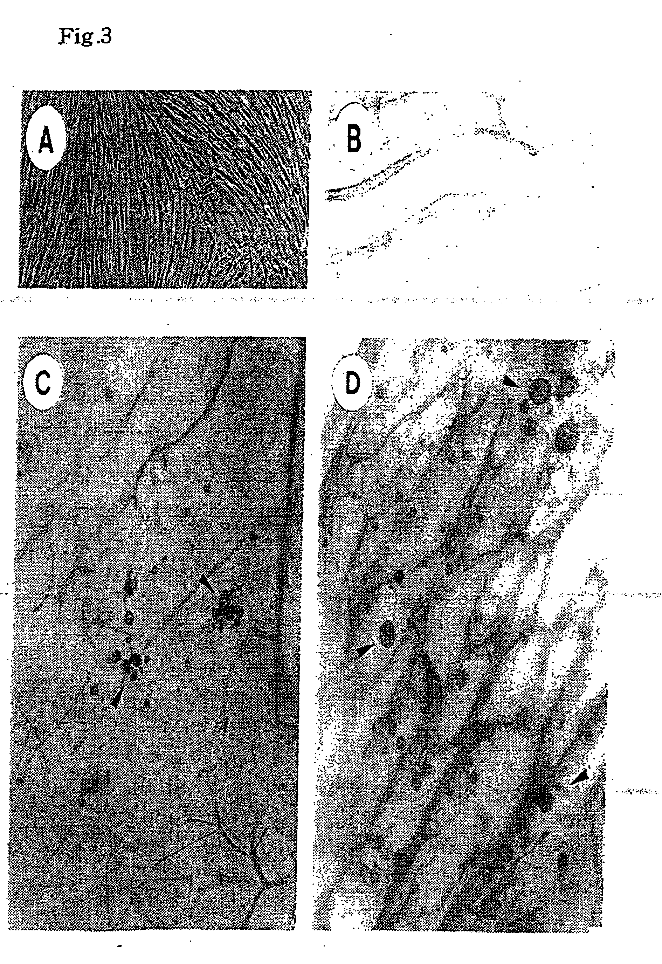

Fig 3 shows cultured human fibroblasts (A), neutralized chitosan sponge (B), neutralized chitosan sponge with cultured human fibroblasts containing no bFGF (C) and neutralized chitosan sponge with cultured human fibroblasts containing bFGF (D). Arrows indicate healthy fibroblasts in the chitosan-matrix fibers.

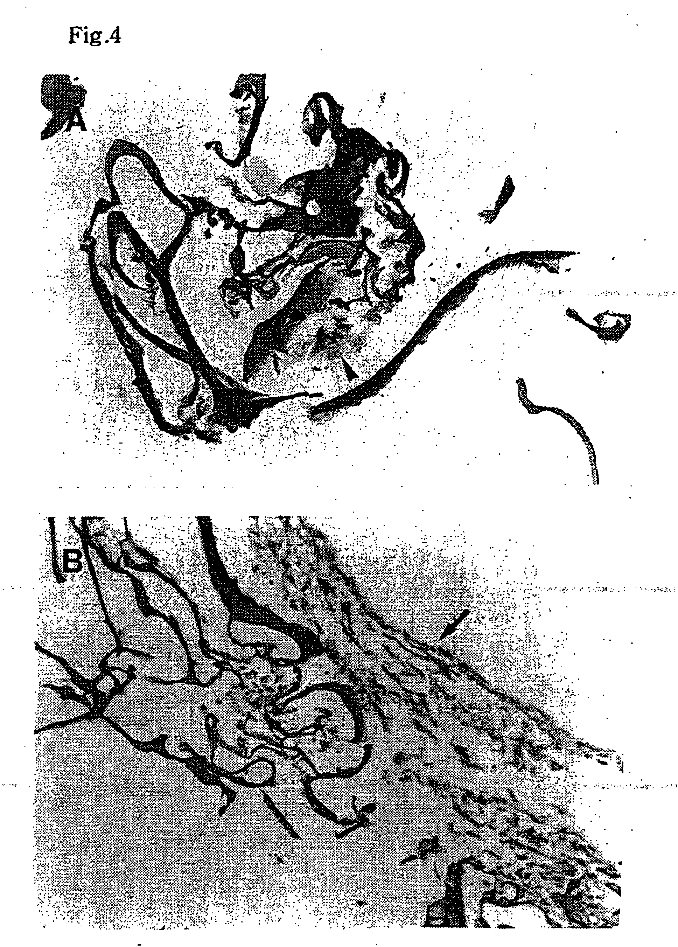

Sections of the neutralized chitosan or the neutralized chitosan/collagen sponge were fixed with 10% formalin/phosphate buffered saline and dehydrated in ethanol. Then, they were washed with xylene and embedded in paraffin overnight. The embedded tissues were sectioned at a thickness of 4 µm using a rotary microtome and the sectioned tissues were stained with hematoxylin-eosin. The neutralized chitosan sponge, particularly, the first few weeks fibroblast growth on the chitosan sponge, tore easily during sectioning. However, the sponge with the fibroblasts cultured for 4 weeks had the improved structural integrity and was sectioned more easily with less disruption (Fig 4: magnification × 200).

In Fig 4(A) (neutralized chitosan sponge), the matrix had little structural integrity and brittle. Human fibroblasts were disrupted during sectioning and rarely shown. Arrows indicate chitosan fibers.

In Fig 4(B) (neutralized chitosan/collagen mixed sponge), the sponge had much more integrity than that in (A). This probably resulted from matrix protein secreted from the cells and collagen fibers. The sponge maintained the structural integrity during sectioning. Arrowheads indicate human fibroblasts and matrix fibers.

### Example 3: Grafting experiment for in vivo application of a bioartificial dermis in Balb/c mouse

### (1) Grafting of an artificial dermis

Balb/c mice were breed in a sterile room. Surgery and grafting were performed in laminar-flow hoods and anesthesia was performed by intraperitoneal injection of 3 mℓ/kg of equithesin. A depilatory was applied onto the dorsal sides of the mice and the hair coat was clipped off and then, the depilated area was disinfected with povidone-iodine and 70% isopropyl alcohol. A full-thickness disk of skin was excised using an autopsy puncture. This excision resulted in approximately 5% total body surface area of skin defect in the experimental animals. Acellular and cellular artificial dermises of the same size as the excision were grafted onto the wound bed and covered with a sterile gauze dressing. To prevent infection, the mice were provided with water supplemented with ampicillin and streptomycin.

The animals were examined for integrity of grafts and healing process everyday and sacrificed after 10 days and 4 weeks. The animals were photographed and tissue was obtained for the following histological analysis.

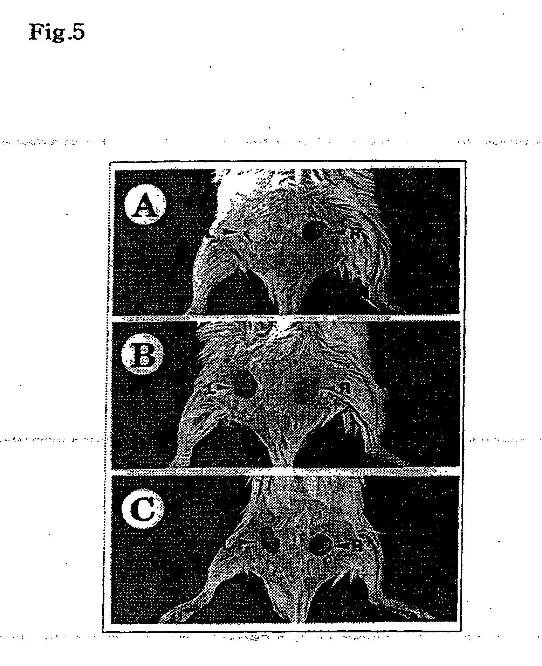

Fig 5 shows chitosan-sponge grafts on the full-thickness excision in Balb/c mouse. In Fig 5(A), the right (R) indicates no graft (control) and the left (L) indicates the neutralized chitosan sponge. In Fig 5(B), R indicates neutralized type-Ip collagen sponge and L indicates neutralized chitosan sponge. In Fig 5(C), R indicates neutralized type-Ip collagen + fibronectin sponge and L indicates neutralized chitosan + type-Ip collagen + fibronectin sponge, respectively. These sponges tightly adhered to wound bed and have no fluid collections.

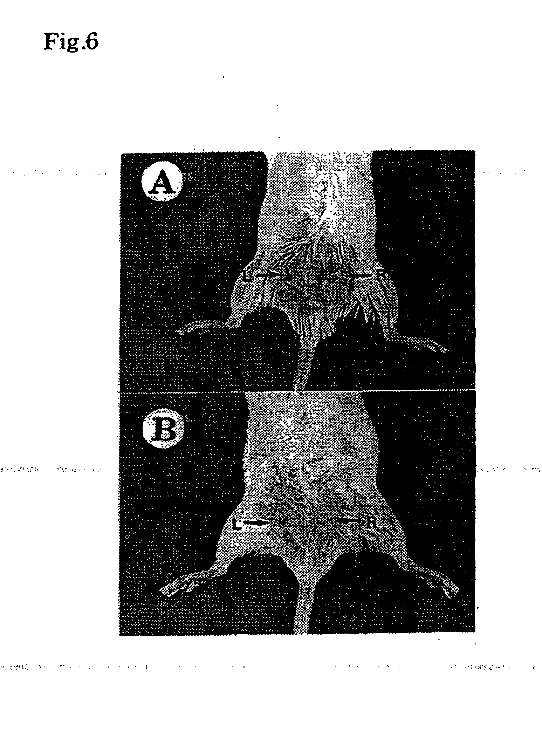

Fig 6 shows graft site in Balb/c mouse 4 weeks after grafting. In (A), R indicates control and L indicates neutralized chitosan sponge. In (B), R indicates neutralized type-Ip collagen sponge, L indicates neutralized chitosan + type-Ip collagen sponge, respectively. It was shown that scar remained in control but the wounds were well healed in the other grafts.

### (2) Preparation of tissue specimen and histological analysis

Tissue specimen from the animal wound was obtained by excision the entire graft with a surrounding rim of normal mouse skin and underlying panniculus carnosus. The sample was fixed with 10% formalin/phosphate buffered saline and embedded in paraffin. Then, it was sectioned according to the same procedure as above and the result was obtained by staining with hematoxylin-eosin.

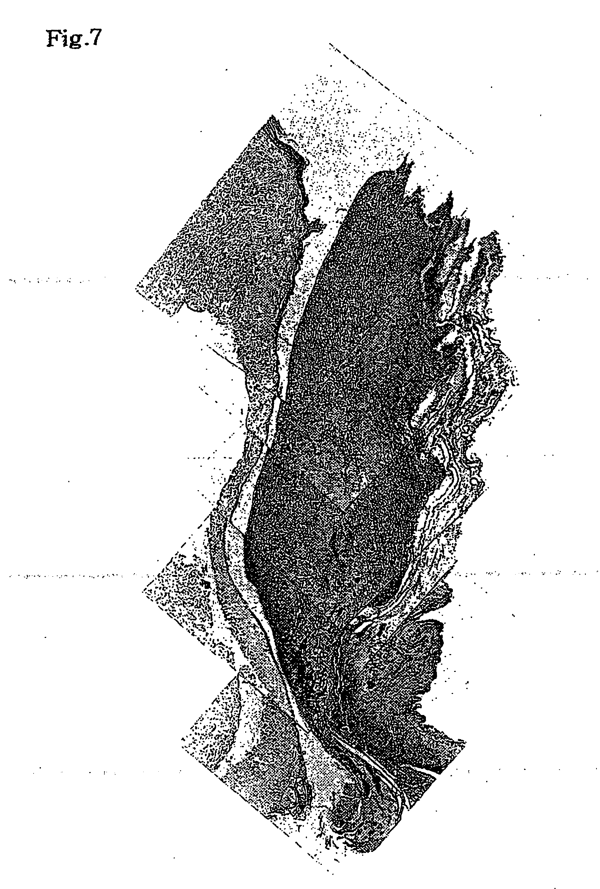

Fig 7 shows gross histology of control biopsied from the dorsolateral region of Balb/c mouse 10 day after grafting (hematoxylin-eosin staining, magnification 100 ×). This area was not fully epithelialized, and did not reconstruct the neodermis and exhibited severe inflammation. This poor healing may be caused by the lack of a dermis.

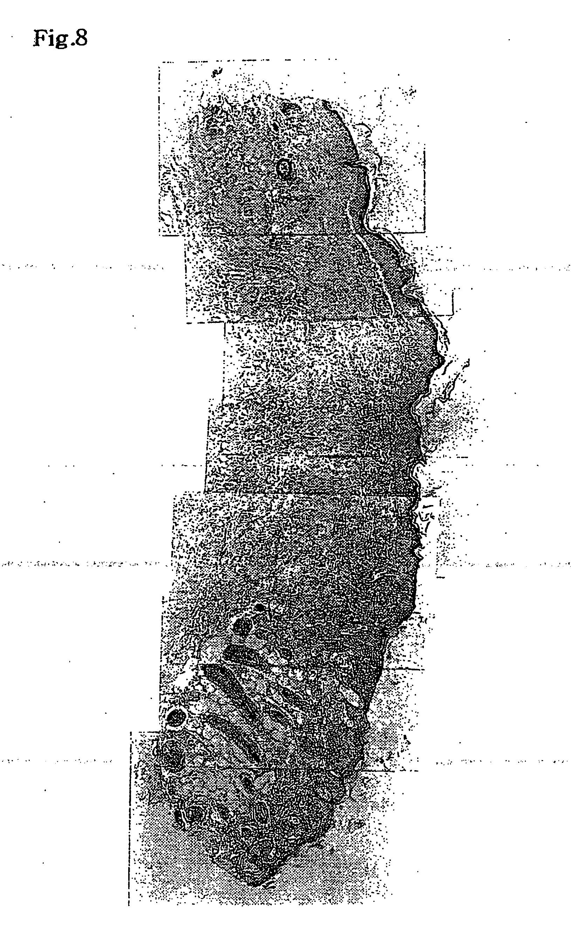

Fig 8 shows gross histology of graft site biopsied from the dorsolateral region of Balb/c mouse grafted with neutralized chitosan sponge 10 days after grafting. Normal skin tissue was found on both sides (determined by many follicular cells which were stained red) and newly regenerated epidermis and dermis were found in the middle. Multi-layered epidermis was formed and dermal cells were migrated into the wound site to reconstruct the neodermis and further, no inflammation was observed.

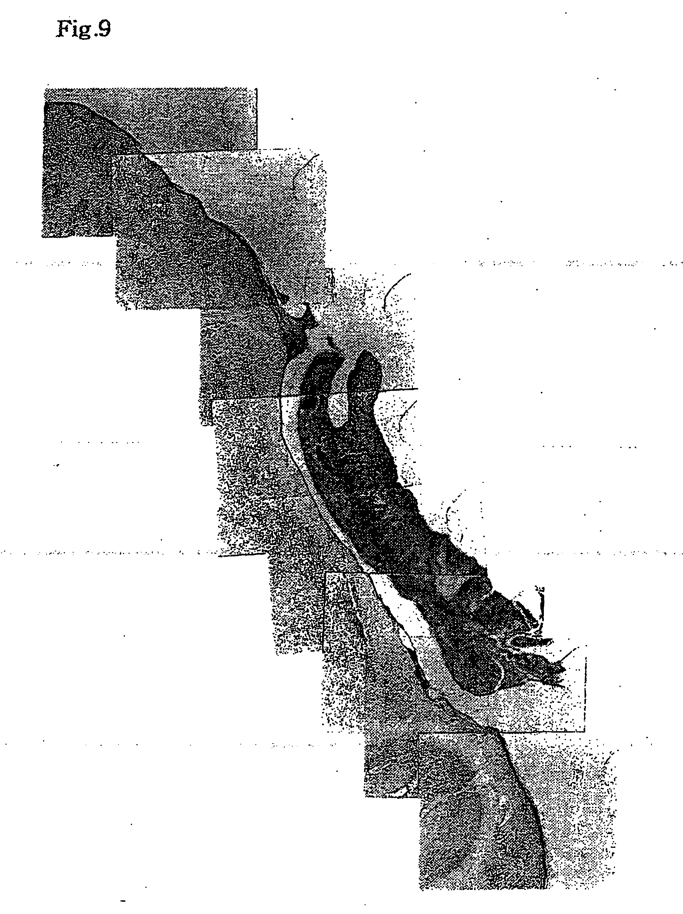

Fig 9 shows gross histology of graft site biopsied from the dorsolateral region of Bal/c mouse grafted with neutralized chitosan sponge containing 50 ng/mℓ of bFGF 10 days after the grafting. In the similar pattern to neutralized chitosan sponge, normal skin tissue was found on both sides and newly regenerated epidermis and dermis were found in the middle. In comparison with neutralized chitosan sponge, a little inflammation was accompanied but on the whole, satisfactory healing effect was obtained.

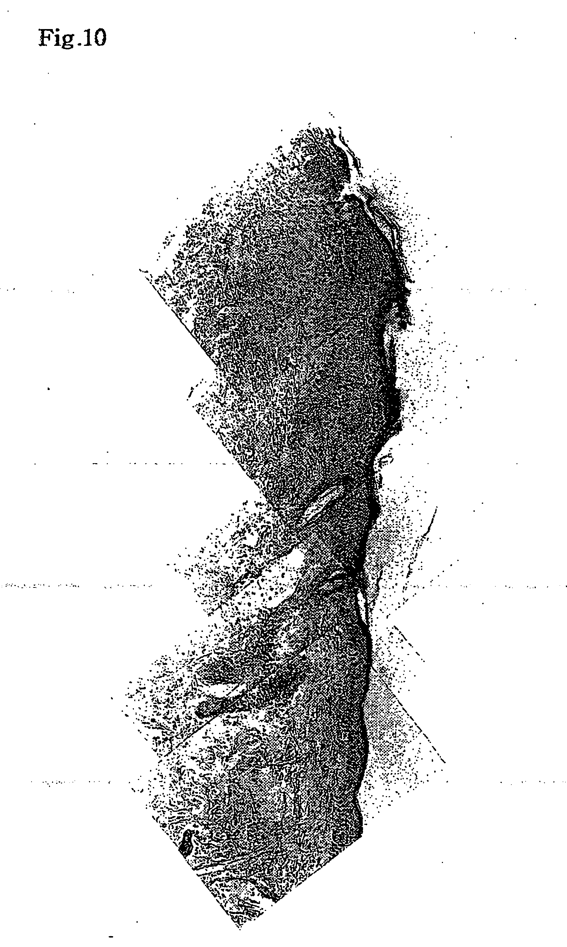

Fig 10 shows gross histology of graft site biopsied from the dorsolateral region of Balb/c mouse grafted with neutralized chitosan/type-Ip collagen mixed sponge 10 days after the grafting. Intact multi-layered epidermis and the reconstructed neodermis were found and further, follicular cells were also found.

### Example 4: Cytotoxicity test of a chitosan derivates in vitro

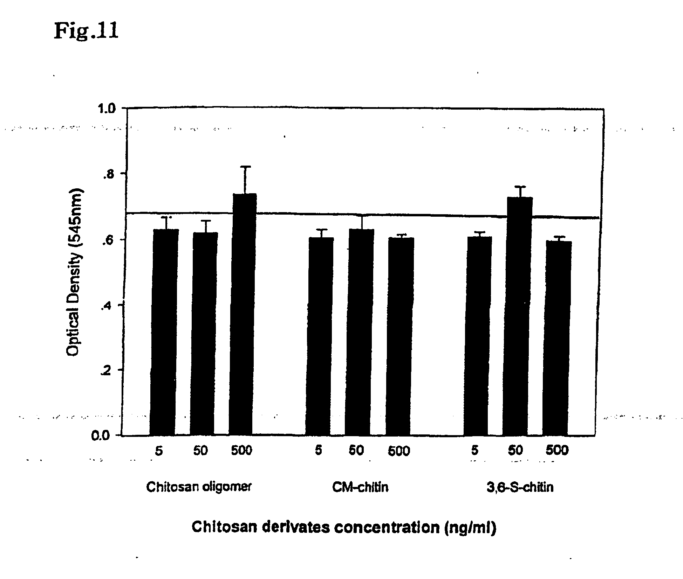

1 × 10⁴ of human dermal fibroblast cells in 96 well plate were cultured in DMEM medium supplemented with 100 µℓ of 10% serum. Then, the cultured cells were treated with 5, 50, and 500 ng/mℓ of chitosan oligomer, CM-chitin and 3,6-S-chitin, respectively. 48 hours after treatment, MTT (3-[4,5-dimethylthiazol-2-yl]-2,5-diphenyltetrazolium bromide; Thiazolyl blue, Sigma Product No. M5655) was added thereto and then, the mixture was allowed to stand for 4 hours. After removal of the culture medium, the resultant mixture was washed with phosphate buffered saline 2-3 times and 100 µℓ of DMSO (dimethylsulfoxide) was added thereto. After chromogenesis, an absorbance was measured at 545 nm on each well using ELISA reader. The results are shown in Fig 11 (line shows control) and the following table 3.

**Table 3.**

| Viability assay result of human dermal fibroblasts treated with chitosan derivates. | | | |
|---|---|---|---|
| | 5 ng/mℓ | 50 ng/mℓ | 500 ng/mℓ |
| chitosan oligomer | 0.629 | 0.618 | 0.733 |
| CM-chitin | 0. 603 | 0.630 | 0.603 |
| 3,6-S-chitin | 0.608 | 0.727 | 0.595 |
| control | 0.677 | | |

As shown above, viability of fibroblast cells treated with the chitosan derivates was in the range of 87.9 to 108.2% of that of control. Therefore, the chitosan derivates were confirmed to have little cytotoxicity *in vitro*. Since the neutralized chitosan and neutralized chitosan/collagen mixed sponges of the present invention are degraded to such chitosan derivates, it is contemplated that they are substantially non-toxic *in vivo*.

Accordingly, the dermal scaffold according to the present invention has excellent wound healing effect by constituting microenvironments suitable for migration and proliferation of fibroblasts and vascular cells surrounding the wound, which is extremely useful as wound healing dressings, and the bioartificial dermis comprising the dermal scaffold and human fibroblasts is particularly useful for healing broad wound sites such as bums wherein migration of the surrounding cells is difficult.

## Claims

1. A dermal scaffold comprising a sponge, consisting of neutralized chitosan.

2. The dermal scaffold of claim 1, **characterized in that** said neutralized chitosan sponge is prepared by neutralization of a chitosan solution with an alkaline solution followed by lyophilization of the neutralized chitosan solution.

3. The dermal scaffold of claim 1, charcterized by further comprising one or more components selected from the group consisting of fibronectin, basic fibroblast growth factor, epidermal growth factor and transforming growth factor-β.

4. A dermal scaffold comprising a neutralized chitosan/collagen mixed sponge wherein the weight ratio of collagen to chitosan is in the range of 1:8 to 1:2 based on the final product, **characterized in that** said neutralized chitosan/collagen mixed sponge is prepared by the method comprising the steps of
a) neutralizing a chitosan solution with an alkaline solution,
b) neutralizing a collagen solution with an alkaline solution,
c) mixing the neutralized chitosan solution prepared in step a) with the neutralized collagen solution prepared in step b), and
d) lyophilizing the mixed solution prepared in step c).

5. The dermal scaffold of claim 4, **characterized by** further comprising one or more components selected from the group consisting of fibronectin, basic fibroblast growth factor, epidermal growth factor and transforming growth factor-β.

6. A dermal scaffold which comprises neutralized chitosan/collagen mixed sponge wherein the weight ratio of collagen to chitosan is in the range of 1:8 to 1:2 based on the final product containing chitosan fabrics.

7. The dermal scaffold of claim 6, **characterized in that** said neutralized chitosan/collagen mixed sponge containing chitosan fabrics is prepared by the method comprising the steps of
a) weaving chitosan fibers into fabrics,
b) neutralizing a chitosan solution with an alkaline solution,
c) applying the neutralized chitosan solution prepared in step b) onto the chitosan fabrics prepared in step a),
d) neutralizing a collagen solution with an alkaline solution, and
e) coating the neutralized chitosan sponge containing chitosan fabrics prepared in step c) with the neutralized collagen solution prepared in step d).

8. The dermal scaffold of claim 6, **characterized by** further comprising one or more components selected from the group consisting of fibronectin, basic fibroblast growth factor, epidermal growth factor and transforming growth factor-β.

9. A bioartificial dermis **characterized in that** human fibroblasts are attached into the dermal scaffold as defined in any one of claims 1 to 3.

10. A bioartificial dermis **characterized in that** human fibroblasts are attached into the dermal scaffold as defined in any one of claims 4 to 5.

11. A bioartificial dermis **characterized in that** human fibroblasts are attached into the dermal scaffold as defined in any one of claims 6 to 8.

## Patentansprüche

1. Dermales Gerüst, umfassend einen Schwamm bestehend aus neutralisiertem Chitosan.

2. Dermales Gerüst nach Anspruch 1, **dadurch gekennzeichnet, dass** der neutralisierte Chitosanschwamm durch Neutralisieren einer Chitosanlösung mit einer alkalischen Lösung gefolgt von Lyophilisieren der neutralisierten Chitosanlösung hergestellt wird.

3. Dermales Gerüst nach Anspruch 1, **dadurch gekennzeichnet, dass** es weiterhin ein oder mehrere Bestandteile, ausgewählt aus der Gruppe bestehend aus Fibronektin, basischem Fibroblasten-Wachstumsfaktor, epidermalem Wachstumsfaktor und transformierendem Wachstumsfaktor β, umfasst.

4. Dermales Gerüst, umfassend einen neutralisierten gemischten Chitosan-/Collagenschwamm, in dem das Gewichtsverhältnis von Collagen zu Chitosan, bezogen auf das Endprodukt, im Bereich von 1:8 bis 1:2 liegt, **dadurch gekennzeichnet, dass** der neutralisierte gemischte Chitosan-/Collagenschwamm hergestellt wird durch das Verfahren, welches die Schritte umfasst:
a) Neutralisieren einer Chitosanlösung mit einer alkalischen Lösung,
b) Neutralisieren einer Collagenlösung mit einer alkalischen Lösung,
c) Mischen der in Schritt a) hergestellten neutralisierten Chitosanlösung mit der in Schritt b) hergestellten neutralisierten Collagenlösung, und
d) Lyophilisieren der in Schritt c) hergestellten gemischten Lösung.

5. Dermales Gerüst nach Anspruch 4, **dadurch gekennzeichnet, dass** es weiterhin ein oder mehrere Bestandteile, ausgewählt aus der Gruppe bestehend aus Fibronektin, basischem Fibroblasten-Wachstumsfaktor, epidermalem Wachstumsfaktor und transformierendem Wachstumsfaktor β, umfasst.

6. Dermales Gerüst, welches neutralisierten gemischten Chitosan-/Collagenschwamm umfasst, in dem das Gewichtsverhältnis von Collagen zu Chitosan, bezogen auf das Endprodukt, im Bereich von 1:8 bis 1:2 liegt, der Chitosangewebe enthält.

7. Dermales Gerüst nach Anspruch 6, **dadurch gekennzeichnet, dass** der neutralisierte gemischte Chitosan-/Collagenschwamm, der Chitosangewebe enthält, hergestellt wird durch das Verfahren, welches die Schritte umfasst:
a) Verweben von Chitosanfasern zu Gewebe,
b) Neutralisieren einer Chitosanlösung mit einer alkalischen Lösung,
c) Aufbringen der in Schritt b) hergestellten neutralisierten Chitosanlösung auf das in Schritt a) hergestellte Chitosangewebe,
d) Neutralisieren einer Collagenlösung mit einer alkalischen Lösung, und
e) Beschichten des in Schritt c) hergestellten neutralisierten Chitosanschwamms, der Chitosangewebe enthält, mit der in Schritt d) hergestellten neutralisierten Collagenlösung.

8. Dermales Gerüst nach Anspruch 6, **dadurch gekennzeichnet, dass** es weiterhin ein oder mehrere Bestandteile, ausgewählt aus der Gruppe bestehend aus Fibronektin, basischem Fibroblasten-Wachstumsfaktor, epidermalem Wachstumsfaktor und transformierendem Wachstumsfaktor β, umfasst.

9. Bioartifizielle Dermis, **dadurch gekennzeichnet, dass** in dem wie in einem der Ansprüche 1 bis 3 definierten dermalen Gerüst menschliche Fibroblasten gebunden sind.

10. Bioartifizielle Dermis, **dadurch gekennzeichnet, dass** in dem wie in einem der Ansprüche 4 bis 5 definierten dermalen Gerüst menschliche Fibroblasten gebunden sind.

11. Bioartifizielle Dermis, **dadurch gekennzeichnet, dass** in dem wie in einem der Ansprüche 6 bis 8 definierten dermalen Gerüst menschliche Fibroblasten gebunden sind.

## Revendications

1. Support dermique comprenant une éponge consistant en de la chitosane neutralisée.

2. Support dermique selon la revendication 1, **caractérisé en ce que** l'éponge à chitosane neutralisée est préparée par la neutralisation d'une solution de chitosane par une solution alcaline suivie d'une lyophilisation de la solution à chitosane neutralisée.

3. Support dermique selon la revendication 1, **caractérisé en ce qu'**il comprend en outre un ou plusieurs composants sélectionnés dans le groupe consistant en la fibronectine, le facteur de croissance fibroblastique de base, le facteur de croissance épidermique et le facteur de croissance de transformation β.

4. Support dermique comprenant une éponge mixte à chitosane/collagène neutralisée, dans laquelle le rapport de poids collagène/chitosane est dans la plage de 1 :8 à 1 :2 sur la base du produit final, **caractérisé en ce que** l'éponge mixte à chitosane/collagène neutralisée est préparée selon le procédé comprenant les étapes consistant à :
a) neutraliser une solution de chitosane avec une solution alcaline,
b) neutraliser une solution de collagène avec une solution alcaline,
c) mélanger une solution à chitosane neutralisée préparée à l'étape a) avec la solution de collagène neutralisée préparée à l'étape b), et
d) lyophiliser la solution mixte préparée à l'étape c).

5. Support dermique selon la revendication 4, **caractérisé en ce qu'**il comprend en outre un ou plusieurs composants sélectionnés dans le groupe consistant en la fibronectine, le facteur de croissance fibroblastique de base, le facteur de croissance épidermique et le facteur de croissance de transformation β.

6. Support dermique comprenant une éponge mixte à chitosane/collagène dans laquelle le rapport de poids collagène/chitosane est dans la plage de 1 :8 à 1 :2 sur la base du produit final, ladite éponge contenant des tissus en chitosane.

7. Support dermique selon la revendication 7, **caractérisé en ce que** l'éponge mixte à chitosane/collagène neutralisée contenant des tissus en chitosane est préparée par la méthode comprenant les étapes de :
a) tissage des fibres de chitosane dans les tissus,
b) neutralisation d'une solution de chitosane avec une solution alcaline,
c) application de la solution de chitosane neutralisée préparée à l'étape b) sur les tissus de chitosane préparés à l'étape a),
d) neutralisation d'une solution de collagène avec une solution alcaline, et
e) trempage de l'éponge de chitosane neutralisée contenant des tissus de chitosane préparé à l'étape c) avec la solution de collagène neutralisée préparée à l'étape d).

8. Support dermique selon la revendication 7, **caractérisé en ce qu'**il comprend en outre un ou plusieurs composants d'un groupe consistant en la fibronectine, le facteur de croissance fibroblastique de base, le facteur de croissance épidermique et le facteur de croissance de transformation β.

9. Derme bioartificiel **caractérisé en ce que** les fibroblastes humains sont attachés au support dermique comme cela est défini dans l'une des revendications 1 à 3.

10. Derme bioartificiel **caractérisé en ce que** les fibroblastes humains sont attachés au support dermique comme cela est défini dans l'une des revendications 4 à 5.

11. Derme bioartificiel **caractérisé en ce que** les fibroblastes humains sont attachés au support dermique comme cela est défini dans l'une des revendications 6 à 8.
